# EUROPEAN PATENT SPECIFICATION

(11) **EP 1 610 745 B1**
(45) Date of publication and mention of the grant of the patent: **06.06.2007**
(21) Application number: 04726992.3
(22) Date of filing: 13.04.2004
(51) Int. Cl.: A61F 13/20, A61F 13/34

(54) **TAMPON, IN PARTICULAR FOR FEMININE HYGIENE**
TAMPON, INSBESONDERE FÜR FRAUEN
TAMPON, EN PARTICULIER POUR L'HYGIENE FEMININE

(30) Priority: 09.04.2003 DE 10316234
(43) Date of publication of application: 04.01.2006
(73) Proprietor: JOHNSON & JOHNSON GmbH, 40474 Düsseldorf (DE)
(72) Inventor: KRÄMER, Robert, 51109 Köln (DE)
(74) Representative: Metten, Karl-Heinz
(86) International application number: PCT/EP2004/003871
(87) International publication number: WO 2004/089264

(56) References cited:
- US-A- 3 863 636
- US-A- 4 328 804

## Description

The invention relates to a tampon, in particular for feminine hygiene, which has a tampon body and a withdrawal tape fastened on the tampon body.

A tampon, in particular a tampon for feminine hygiene, is provided with a withdrawal tape in order for it to be possible for the used tampon easily to be pulled out of, and removed from, the user's vaginal opening. The withdrawal tape is anchored firmly in the tampon body and usually comprises one or more threads which have a length of approximately 10 to 15 cm, measured from the rear end of the tampon body. In a number of configurations, the withdrawal tape comprises two withdrawal threads which are knotted together at their end.

It has been found, in practice, that in particular young women who play sports want a tampon with a shorter and as far as possible "invisible" withdrawal tape. There are various reasons for this.

While urinating, it would be extremely difficult and impractical to grip the withdrawal tape in order to keep it away from the flow of urine, so that most women accept that the withdrawal tape will become wet and saturated with liquid when they urinate. This may both render the tampon uncomfortable to wear, as a result of the damp withdrawal tape, and be a source of infection.

A recent test, furthermore, has established that, during menstruation, women choose to use tampons for sport and during leisure time and holidays because tampons afford largely invisible protection, which was one of the main concerns of the users. For example more than 50% of those taking part in the tests gave this as a desirable effect of tampons used as protection during menstruation. It has also been found that a large number of users find the visibility of the withdrawal tape in swimming pools and saunas and on the beach both unpleasant and embarrassing. On account of its customary length of approximately 10 to 15 cm, measured from the rear end of the tampon, a withdrawal tape may be visible when the tampon is worn, e.g. for swimming, in the sauna, etc.

There is thus a need for tampons for feminine hygiene which have a short, as far as possible invisible withdrawal tape, in particular for use in specific situations, such as for sport, in the sauna, on the beach, etc. On the other hand, many women prefer, in most situations, tampons with a withdrawal tape of customary length, because this is easier to grip and to handle in order to pull the tampon out of the vaginal opening.

US Patent 6,312,419 deals with the theme of short and extensible withdrawal tapes for tampons, the problem on which this patent is based being that the standard length of the withdrawal tapes for tampons is considered too short and thus a lengthening device which can be fitted on the withdrawal tape is proposed for the better handling of the withdrawal tape.

US Patent 6,142,489 deals with the problem of the possibility of a withdrawal tape being saturated with liquid when one urinates, and proposes the solution of coating the withdrawal tape. This specification, however, does not deal with the length of the withdrawal tape.

The object of the invention is to specify a tampon, in particular for feminine hygiene, which allows a user to make a selection between a shorter withdrawal tape and a longer withdrawal tape. This object is achieved by a tampon having the feature of Claim 1. The invention also proposes methods of producing such tampons.

The tampon according to the invention comprises a tampon body and a withdrawal tape fastened on the tampon body. The withdrawal tape is configured such that its effective length can be changed preferably before use, but also during use. In particular, the withdrawal tape is formed with at least one loop or a fold which reduces the effective length of the withdrawal tape and can be released in order to increase the effective length of the withdrawal tape. Effective length refers to the extent of the withdrawal tape, measured from the rear end of the tampon body, which is available for the user to grip.

The tampon according to the invention can be used with the shortened withdrawal tape, that is to say with the loop or fold, which has the advantage that only a short effective withdrawal tape extends from the rear end of the tampon body, which short tape is barely visible, if at all, during use and can absorb less urine and menstrual fluid. If the free end of the withdrawal tape is pulled before use, it is possible, by virtue of the pulling action, to release the loop or fold and thus increase the effective length of the withdrawal tape, with the result that the same tampon also gives the option of a withdrawal tape of standard length, which is easier to grip and to handle when the tampon is to be removed.

The invention proposes two different preferred embodiments of the tampon. In one embodiment, the withdrawal tape has one end connected to the tampon body and its other, free end knotted to form a loop which can be released by virtue of the free end being pulled. This "knotted" embodiment has the advantage that the production equipment does not have to be changed in any way during the production of the tampon, and that all that is required is for steps to be taken in order to knot the withdrawal tape to form a loop. There are a large number of standard knots which can be used in this embodiment of the invention, it being the intention for the loop to be formed such that it releases by virtue of the free end of the withdrawal tape being pulled, but cannot be released by virtue of the loop itself being pulled.

Examples of knots for forming the loop are constituted by a loop in the manner of a half-loop or in the manner of a Windsor knot. The person skilled in the art will be able to provide other types of knot.

In the second preferred configuration of the invention, the withdrawal tape is arranged partly within the tampon body and is positioned and retained in at least one fold therein. During the production of the tampon, the withdrawal tape, rather than being led out of the tampon over the shortest route, is positioned in one or more folds parallel to, obliquely in relation to or transversely to the longitudinal extent of the tampon and is integrated and retained in the tampon body by virtue of the latter being rolled and pressed. The folds of the withdrawal tape in the tampon body are designed such that they can be released by virtue of the free end of the withdrawal tape being pulled, in order to increase the effective length of the withdrawal tape outside the tampon.

This configuration has the advantage that the equipment for producing the knot in the end of the withdrawal tape does not have to be changed in any way, although a slight modification in the production process of the tampon is necessary.

The invention is explained in more detail hereinbelow by way of the preferred embodiments and with reference to the drawings, in which:
- Figures 1 to 6: show schematic illustrations of the production and use of a tampon according to a first embodiment of the invention; and
- Figures 7 to 10: show schematic illustrations of the steps for producing a tampon according to a second embodiment of the invention.

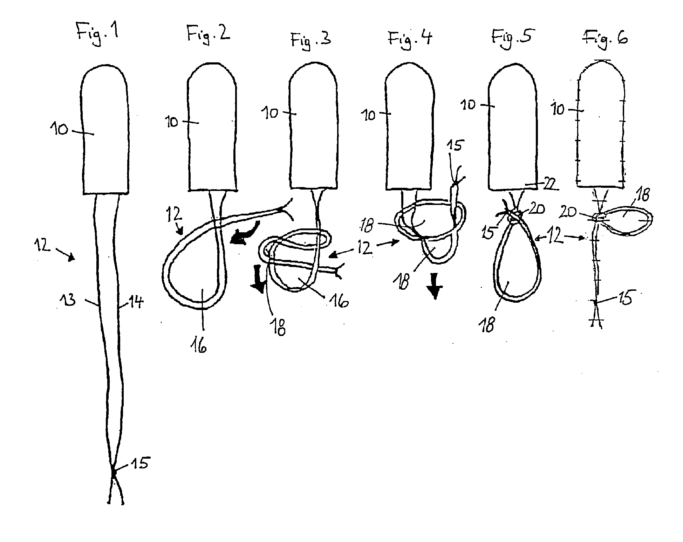

Figure 1 shows a tampon comprising a tampon body 10 and a withdrawal tape 12 which is formed from two threads 13, 14 which are connected by a knot 15 at their free end.

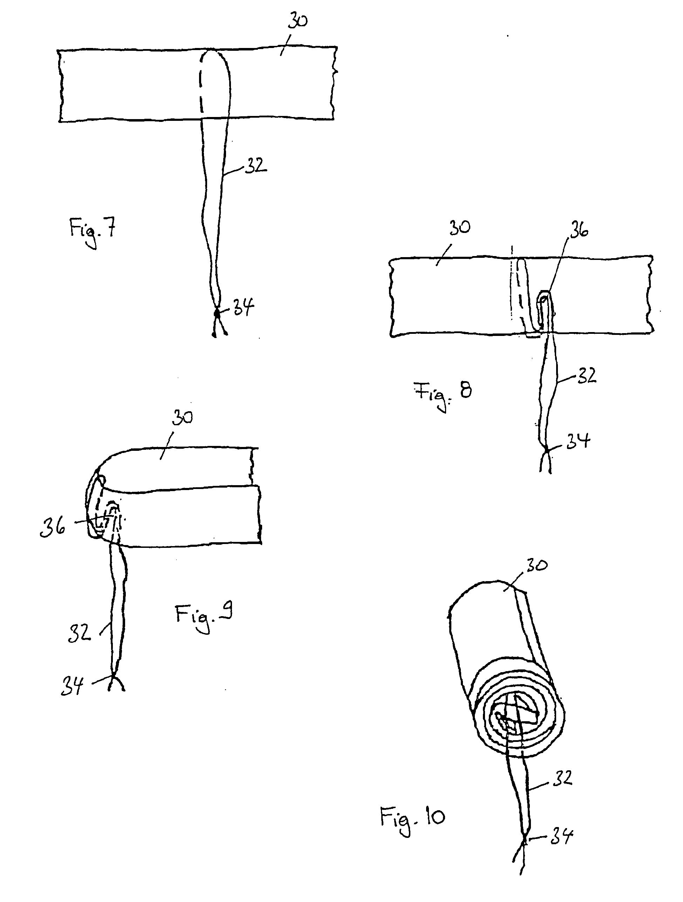

Figures 2 to 6 illustrate schematically how, in the first preferred embodiment of the invention, the effective length of the withdrawal tape 12 is shortened by knotting a loop and how the loop can be lengthened again. As is shown in Figure 2, in a first step, the withdrawal tape 12 forms a loop 16. In the next step, which is illustrated in Figure 3, a further loop 18 is formed, this being pulled through the first loop 16. In the next step, which is shown in Figure 4, the second loop 18 is pulled away from the tampon body 10, while the first loop 16 and the knot 15 of the withdrawal tape 12 are simultaneously retained firmly in order to form the relatively large loop 18 which is shown in Figure 5, and is retained in the vicinity of the rear end 22 of the tampon body 16 by a knot 20 formed by the first loop 16.

Forming the loop 18 produces a reduced effective length of the withdrawal tape 12, measured from the rear end 22 of the tampon body 10, this length being approximately equal to half the length of the straightened-out withdrawal tape 12 from Figure 1.

It is easily possible, by virtue of the free end of the withdrawal tape 12 being pulled, at the knot 15, for the loop 18 to be released again in order to bring the withdrawal tape 12 back to its maximum length.

The first embodiment of the tampon according to the invention thus specifies a solution for a variable-length withdrawal tape which is particularly straightforward to realize and easy to handle. It is immediately obvious to the user that the knotted loop 18 can be used as a short withdrawal tape or alternatively can be released in order to form a longer withdrawal tape.

A further embodiment of the invention is described hereinbelow with reference to Figures 7 to 10.

Figure 7 shows, schematically, part of a fabric tampon band 30 and a withdrawal tape 32 which is positioned around the fabric tampon band 30, transversely to the longitudinal extent thereof, and is knotted at its ends. The end knot is designated 34. As is shown in Figure 7, the withdrawal tape 32 extends transversely to the fabric tampon strip 30 and laterally beyond the latter.

The arrangement shown in Figure 7 corresponds to a standard arrangement of fabric tampon strip and withdrawal tape for producing tampons in accordance with which the withdrawal tape is positioned around a fabric tampon strip and the latter is then rolled and pressed in order to form the tampon.

According to the invention, the withdrawal tape 32 is positioned in at least one fold 36 before the fabric tampon band 30 is rolled and pressed, it being possible for this fold 36 to be positioned parallel to, obliquely in relation to or transversely to the transverse extent of the fabric tampon band 30. In contrast to the illustration of Figure 8, it is also possible for the withdrawal tape 32 to be positioned in more than one fold 36.

As is shown in Figure 9, the fabric tampon band 30 is then folded together, with the result that the withdrawal tape 32 with the fold 36 comes to rest in the fold of the folded fabric tampon band 30. In a following tampon-production step, which is shown in Figure 10, the folded-together fabric tampon band 30, with the fold 36 therein, is rolled and pressed in order to form the finished tampon. The operations of folding the fabric tampon band 30 together, this being shown in Figure 9, and of rolling and pressing the tampon may correspond, in principle, to the conventional method for producing tampons, with the exception that the withdrawal tape 32, rather than being led away rectilinearly from the tampon, transversely to the longitudinal extent of the fabric tampon band 30, is positioned in at least one fold 36.

The withdrawal tape 32 may be folded, for example, such that the withdrawal tape can be lengthened from a short, effective length of approximately 5 to 8 cm, in particular 6.5 cm, outside the tampon body to an extended effective length of 10 to 15 cm, in particular approximately 13.5 cm. During use, the user can decide whether to use the tampon with the short withdrawal tape, in the state provided by the manufacturer, or whether, before using the tampon, to pull the withdrawal tape 32 out of the tampon until it reaches its extended length, in order that the tampon can be used with a longer withdrawal tape.

Tests have shown that, in the second embodiment of the invention, a force of 0.4 to 2.2 N is necessary in order to pull the withdrawal tape 32 out of the tampon body. Tampons with a weight of between 2.4 g and 2.7 g and a stability in the region of approximately 20 N to 35 N were used in these tests. These tests have also shown that the force for pulling the withdrawal tape out is dependent on the arrangement of the fold 36 on the fabric tampon band 30. With the fold 36 arranged parallel to the withdrawal tape 32, as is shown in Figure 8, the force which was necessary in order to pull the withdrawal tape out of the tampon body was slightly lower than in the case of the withdrawal tape 32 being folded obliquely or laterally in relation to the transverse direction of the fabric band 30.

The features disclosed in the above description, in the claims and in the drawings may be significant both individually and in any desired combination in order to realize the various embodiments of the invention.

### List of designations:

- Tampon body: 10
- Withdrawal tape: 12
- Threads: 13, 14
- Knot(s): 15
- First loop: 16
- Second loop: 18
- Knot(s): 20
- Rear end: 22
- Fabric band of tampon: 30
- Withdrawal tape: 32
- End knot(s): 34
- Fold: 36

## Claims

1. Tampon, in particular for feminine hygiene, comprising a tampon body (10) and a withdrawal tape (12) which is fastened on the tampon body (10) and is configured such that its effective length can be changed before or during use, wherein the withdrawal tape (12; 32) is formed with at least one loop (18) which reduces the effective length of the withdrawal tape (12) and can be released in order to increase the effective length of the withdrawal tape (12),
**characterized in that** the withdrawal tape (12) has a first end connected to the tampon body (10) and a second end knotted to form a knot (20), a loop (18) and a free end (15) extending from the knot (20), where the loop (18) can be released by virtue of the free end (15) being pulled.

2. Tampon according to claim 1, **characterized in that** the loop (18) is formed such that it does not release by virtue of the loop (18) being pulled.

3. Tampon according to claim 1 or 2, **characterized in that** the loop (18) is formed in the manner of a half loop.

4. Tampon according to claim 1 or 2 **characterized in that** the loop (18) is formed in the manner of a Windsor knot.

5. Tampon, in particular for feminine hygiene, comprising a tampon body (10) and a withdrawal tape (32) which is fastened on the tampon body (10) and is configured such that its effective length can be changed before or during use, wherein the withdrawal tape (32) is formed with at least one fold (36) which reduces the effective length of the withdrawal tape (32) and can be released in order to increase the effective length of the withdrawal tape (32), where the withdrawal tape (32) is arranged partly within the tampon body (30) and is positioned in the at least one fold (36) therein, **characterized in that** a portion of the withdrawal tape is retained in the form of the at least one fold (36) between adjacent parts of the tampon band (30) by virtue of the latter being rolled and pressed, where said at least one fold (36) of the withdrawal tape (32) within the tampon body (30) can be released by virtue of the free end of the withdrawal tape (32) being pulled, in order to increase the effective length of the withdrawal tape (32) outside the tampon.

6. Method of producing a tampon according to one of claims 1 to 4, **characterized in that** the withdrawal tape (12) is positioned around a fabric tampon band such that it extends transversely to the fabric tampon band, and laterally beyond the latter, **in that** the fabric tampon band is rolled and pressed in order to form the tampon body (10) with the withdrawal tape (12) fastened therein, and **in that** the withdrawal tape (21) is knotted outside the tampon body (10) to form a knot (20), a loop (18) and a free end (15) extending from the knot (20), where the loop (18) can be released by virtue of the free end (15) being pulled.

7. Method of producing a tampon according to claim 5, **characterized in that** a withdrawal tape (32) is positioned around a fabric tampon band (30) such that it extends transversely to the fabric tampon band (30), and laterally beyond the latter, that part of the withdrawal tape (32) which extends laterally beyond the fabric tampon band (30) is folded back partly onto the fabric tampon band (30), and the fabric tampon band is rolled and pressed in order to form the tampon body with the withdrawal tape (32) fastened therein and to retain the folded-back part of the withdrawal tape (32) in the tampon.

8. Method according to claim 7, **characterized in that** the withdrawal tape (32) is folded back onto the fabric tampon band (30) parallel to, obliquely in relation to or perpendicularly to the transverse direction of the fabric tampon band.

## Patentansprüche

1. Tampon, im besonderen für weibliche Hygiene, umfassend einen Tamponkörper (10) und ein Rückholbändchen (12), das am Tamponkörper (10) befestigt ist und so gestaltet ist, daß seine effektive Länge vor oder während des Gebrauchs verändert werden kann, wobei das Rückholbändchen (12; 32) mit zumindest einer Schlinge (18) ausgebildet ist, welche die effektive Länge des Rückholbändchens (12) verkürzt und die gelöst werden kann, um die effektive Länge des Rückholbändchens (12) zu verlängern,
**dadurch gekennzeichnet, daß** das Rückholbändchen (12) ein erstes Ende aufweist, das mit dem Tamponkörper (10) verbunden ist, und ein zweites Ende, das verknotet ist, um einen Knoten (20) auszubilden, eine Schlinge (18) und ein freies Ende (15), welches sich aus dem Knoten (20) heraus erstreckt, wobei die Schlinge (18) gelöst werden kann, indem an dem freien Ende (15) gezogen wird.

2. Tampon nach Anspruch 1, **dadurch gekennzeichnet, daß** die Schlinge (18) so ausgebildet ist, daß sie sich nicht löst, wenn an der Schlinge (18) gezogen wird.

3. Tampon nach Anspruch 1 oder 2, **dadurch gekennzeichnet, daß** die Schlinge (18) in Form einer Halb-Schlinge ausgebildet ist.

4. Tampon nach Anspruch 1 oder 2, **dadurch gekennzeichnet, daß** die Schlinge (18) in Form eines Windsor-Knotens ausgebildet ist.

5. Tampon, im besonderen für weibliche Hygiene, umfassend einen Tamponkörper (10) und ein Rückholbändchen (32), das am Tamponkörper (10) befestigt ist und so gestaltet ist, daß seine effektive Länge vor oder während des Gebrauchs verändert werden kann, wobei das Rückholbändchen (32) mit zumindest einer Faltung (36) ausgebildet ist, welche die effektive Länge des Rückholbändchens (32) verkürzt und gelöst werden kann, um die effektive Länge des Rückholbändchens (32) zu verlängern, wobei das Rückholbändchen (32) zum Teil innerhalb des Tamponkörpers (30) angeordnet ist und in der zumindest einen Faltung (36) darin positioniert ist, **dadurch gekennzeichnet, daß** ein Abschnitt des Rückholbändchens in Form der zumindest einen Faltung (36) zwischen benachbarten Teilen des Tamponstreifens (30) zurückgehalten wird, wenn letzterer gerollt und gepreßt wird, wobei die zumindest eine Faltung (36) des Rückholbändchens (32) innerhalb des Tamponkörpers (30) gelöst werden kann, wenn am freien Ende des Rückholbändchens (32) gezogen wird, um die effektive Länge des Rückholbändchens (32) außerhalb des Tampons zu verlängern.

6. Verfahren für die Herstellung eines Tampons nach einem der Ansprüche 1 bis 4, **dadurch gekennzeichnet, daß** das Rückholbändchen (12) rund um einen Gewebe-Tamponstreifen so positioniert wird, daß es sich transversal zum Gewebe-Tamponstreifen und seitlich über letzteren hinaus erstreckt, daß der Gewebe-Tamponstreifen gerollt und gepreßt wird, um den Tamponkörper (10) mit dem darin befestigten Rückholbändchen (12) auszubilden, und daß das Rückholbändchen (21) außerhalb des Tamponkörpers (10) verknotet wird, um einen Knoten (20), ein Schlinge (18) und ein freies Ende (15) auszubilden, welches sich aus dem Knoten (20) heraus erstreckt, wobei die Schlinge (18) gelöst werden kann, indem am freien Ende (15) gezogen wird.

7. Verfahren für die Herstellung eines Tampons nach Anspruch 5, **dadurch gekennzeichnet, daß** ein Rückholbändchen (32) rund um einen Gewebe-Tamponstreifen (30) so positioniert wird, daß es sich transversal zum Gewebe-Tamponstreifen (30) und seitlich über letzteren hinaus erstreckt, daß der Teil des Rückholbändchens (32), der sich seitlich über den Gewebe-Tamponstreifen (30) hinaus erstreckt, zum Teil auf den Gewebe-Tamponstreifen (30) zurückgefaltet wird, und der Gewebe-Tamponstreifen gerollt und gepreßt wird, um den Tamponkörper mit dem darin befestigten Rückholbändchen (32) auszubilden und den zurückgefalteten Teil des Rückholbändchens (32) im Tampon zurückzuhalten.

8. Verfahren nach Anspruch 7, **dadurch gekennzeichnet, daß** das Rückholbändchen (32) auf den Gewebe-Tamponstreifen (30) parallel zur, schräg im Bezug auf oder senkrecht zur transversalen Richtung des Gewebe-Tamponstreifens zurückgefaltet wird.

## Revendications

1. Tampon, en particulier pour l'hygiène féminine, comprenant un corps de tampon (10) et une bande de retrait (12) qui est fixée sur le corps de tampon (10) et est configurée de sorte que sa longueur effective peut être modifiée avant ou pendant l'utilisation, dans lequel la bande de retrait (12 ; 32) est formée avec au moins une boucle (18) qui réduit la longueur effective de la bande de retrait (12) et peut être libérée afin d'augmenter la longueur effective de la bande de retrait (12),
**caractérisé en ce que** la bande de retrait (12) a une première extrémité raccordée au corps de tampon (10) et une seconde extrémité nouée pour former un noeud (20), une boucle (18) et une extrémité libre (15) s'étendant à partir du noeud (20), dans lequel la boucle (18) peut être libérée en vertu de l'extrémité libre (15) qui est tirée.

2. Tampon selon la revendication 1, **caractérisé en ce que** la boucle (18) est formée de sorte qu'elle ne se relâche pas en vertu du fait que la boucle (18) est tirée.

3. Tampon selon la revendication 1 ou 2, **caractérisé en ce que** la boucle (18) est formée à la manière d'une demi-boucle.

4. Tampon selon la revendication 1 ou 2, **caractérisé en ce que** la boucle (18) est formée à la manière d'un noeud de cravate.

5. Tampon en particulier pour l'hygiène féminine comprenant un corps de tampon (10) et une bande retrait (32) qui est fixée sur le corps de tampon (10) et est configurée de sorte que sa longueur effective peut être modifiée avant ou pendant l'utilisation, dans lequel la bande de retrait (32) est formée avec au moins un pli (36) qui réduit la longueur effective de la bande de retrait (32) et peut être libérée afin d'augmenter la longueur effective de la bande de retrait (32), où la bande de retrait (32) est agencée partiellement dans le corps de tampon (30) et est positionnée dans le au moins un pli (36) à l'intérieur de celui-ci, **caractérisé en ce qu'**une partie de la bande de retrait est au moins retenue sous la forme du au moins un pli (36) entre les parties adjacentes de la bande de tampon (30) en vertu du fait que cette dernière est enroulée et comprimée, où ledit au moins un pli (36) de la bande de retrait (32) à l'intérieur du corps de tampon (30) peut être libéré en vertu de l'extrémité libre de la bande de retrait (32) qui est tirée, afin d'augmenter la longueur effective de la bande de retrait (32) à l'extérieur du tampon.

6. Procédé de production d'un tampon selon l'une quelconque des revendications 1 à 4, **caractérisé en ce que** la bande de retrait (12) est positionnée autour d'une bande de tampon en tissu de sorte qu'elle s'étend transversalement par rapport à la bande de tampon en tissu, latéralement au-delà de cette dernière, **en ce que** la bande de tampon en tissu est enroulée et comprimée afin de former le corps de tampon (10) avec la bande de retrait (12) fixée à l'intérieur de celui-ci, et **en ce que** la bande de retrait (21) est nouée à l'extérieur du corps de tampon (10) pour former un noeud (20), une boucle (18) et une extrémité libre (15) s'étendant à partir du noeud (20), où la boucle (18) peut être libérée en vertu de l'extrémité libre (15) qui est tirée.

7. Procédé de production d'un tampon selon la revendication 5, **caractérisé en ce que** la bande de retrait (32) est positionnée autour d'une bande de tampon en tissu (30) de sorte qu'elle s'étend de manière transversale par rapport à la bande de tampon en tissu (30) et latéralement au-delà de cette dernière, **en ce qu'**une partie de la bande de retrait (32) qui s'étend latéralement au-delà de la bande de tampon en tissu (30) est repliée partiellement sur la bande de tampon en tissu (30), et la bande de tampon en tissu est enroulée et comprimée afin de former le corps de tampon avec la bande de retrait (32) fixée à l'intérieur de celui-ci et afin de retenir la partie repliée de la bande de retrait (32) dans le tampon.

8. Procédé selon la revendication 7, **caractérisé en ce que** la bande retrait (32) est repliée sur la bande de tampon en tissu (30) parallèle à, de manière oblique par rapport à ou perpendiculairement à la direction transversale de la bande de tampon en tissu.
